# EUROPEAN PATENT APPLICATION

(11) **EP 1 598 415 A1**
(43) Date of publication of application: **23.11.2005**
(21) Application number: 04252944.6
(22) Date of filing: 20.05.2004
(51) Int. Cl.: C12M 3/00, C12N 5/06, G01N 35/00, C12M 1/36

(54) **Smart cell culture**

(71) Applicant: The Automation Partnership (Cambridge) Limited, Royston, Hertfordshire SG8 5WY (GB)
(72) Inventor: Drake, Rosemary Ann Lucy, Royston Hertfordshire SG8 8SN (GB); Oakeshott, Robert Bernard Simon, Cambridge CB2 2HU (GB)
(74) Representative: Brunner, Michael John

(57) **Abstract**

There is provided a system for cultivating cells in cell culture vessels. The system includes a liquid handling module, an incubator module, a testing module for performing measurement upon cells and/or media and generating output data, a manipulator module, and a workflow management module for controlling the execution of processes within the system. The workflow management module includes a smart decision making means for selectively processing cells and/or media in accordance with any of process definitions, operational rules and output data from the testing module. The workflow management module also includes manipulator control means and liquid handling control means for controlling the operation of the manipulator module and the liquid handling module respectively in accordance with any of the process definitions, operational rules and decisions from the decision making means.

## Description

### Introduction

The invention relates to a system and method for cultivating cells. In particular, the invention relates to a cell cultivating system that implements an intelligent decision making process.

Cells and cell-derived products, such as proteins, are vital tools in many areas of drug discovery and development in the genomics, biotechnology and pharmaceutical industries. Genetically modified cell lines and proteins are used to help elucidate the process of disease, to discover appropriate targets for drug therapy, to study protein structures and as reagents in assays for screening libraries of chemical compounds to identify lead compounds suitable for further development. The pharmacokinetic, metabolic and toxicological properties of chemical compounds are also studied by means of cell based assays. In addition, cells and proteins derived from cells form the basis of many modern therapies, including therapeutic antibodies.

These different activities require the efficient culture of cells of many types and morphologies. The types of cells that are used to support these activities range include mammalian cells, insect cells, yeasts and bacteria. Each type has particular characteristics and the researcher selects the appropriate cell type accordingly. For example, bacteria grow rapidly and are easy to culture, mammalian cells grow more slowly but are able to perform post-translational modification of proteins and have complex signalling pathways that can be interrogated in functional assays.

Each cell type and cell line (specific type or strain of cell) will have specific requirements if they are to grow and express protein, or other characteristic of interest, efficiently. These requirements include provision of the right environment e.g. temperature, oxygen, pH, and nutrient mix "media" (salts, glucose, lipids, amino acids, vitamins, hormones, growth factors etc.). They may also include selective agents (to suppress unmodified cells and ensure that only modified cell lines grow and divide) and inducer (as a signal for protein expression). Some cells such as mammalian cells are far more stringent in their requirements, others such as bacteria can thrive under a wide range of environmental conditions on simple defined media.

In order that the researcher has cells or protein of the right quality for their research, they must provide the appropriate growth conditions for the cells. Often significant amounts of routine manual work are necessary to maintain the correct nutrient and gas mix, remove waste products and provide the right temperature for incubation, in order to sustain a constant supply of cells.

Conventional cell culture techniques require manual performance of a series of steps (tasks). The steps may include seeding, re-feeding, harvesting, expansion, checking cell growth, liquid disposal, and limiting dilution.

Whether the techniques used are manual or automated, a variety of types of vessels, lids, pipettes, and tubes are used in the culture of cells: the term "labware" is the generic term. Examples of particular types of vessels include reservoirs, flasks, bottles, plates (comprising fixed arrays of fluidly separated dishes - i.e. multiwell dishes) and culture vessel blocks (CVBs). Lids may be provided for each vessel. They may alternatively cover more than one vessel, exposing the contents of more than one vessel when the lid is removed.

### Known cell culture techniques - Protein production

Heterologous gene expression for protein production, and purification of that protein, are essential first steps in many areas of research and development for drug discovery. There has been a recent shift in focus in drug discovery to the identification of appropriate "drugable" targets, that is proteins and receptors.

As target analysis becomes both faster and increasingly done in parallel, there is a rise in the demand for proteins. The consequences are that many more genes and subgenomic fragments, with a range of attached ancillary sequences, need to be expressed as proteins and investigated.

*Escherichia coli,* yeast and insect cells infected with baculovirus are routinely used for heterologous protein expression because their culture is simple and rapid. Within a few hours, or at most a few days, usable levels of protein can be obtained, and then the best constructs and conditions selected for subsequent scale-up.

Although it is feasible for an individual to create and culture small numbers of strains in parallel, it becomes difficult, if not impossible, to manage and control the process as the total increases to tens or hundreds in one experiment. Using manual techniques is time consuming and labour intensive - so compromises inevitably have to be made. Experiments are performed serially, not in a highly parallel way, so they take longer. Experimental strategies that require exploration of a wide range of process parameters are not feasible or are limited by staff working practices and working hours. Handling greater numbers brings associated problems of tracking the samples through all process steps.

The steps of protein expression and production of recombinant organisms, their culture, and the subsequent protein extraction and purification have become significant bottlenecks for many laboratories. Protein production is frequently rate limiting - it can take months to produce the quantity and quality required, and require several iterations. Often only limited quantities of protein are available for experimentation. Limited protein availability hampers structural biology research as well as other activities, such as screening. Limited protein availability also means that it takes longer to solve the target protein structure, so that structural information is not available when it would be advantageous for development. Decisions on which compounds to take forward into lead optimisation are delayed.

### Known cell culture techniques - Stable cell line generation

To support some types of drug discovery research and for the production of therapeutic proteins, it is necessary to develop new cell lines that have been modified to have specific characteristics, such as expression of a protein or receptor. For some experiments transient modification is acceptable, but it is often necessary to create stable cell lines.

The process of stable mammalian cell lines generation can take several weeks to months, before a clone with the required combination of properties is available. During this period, cells are generally cultured at a small scale in multiwell plates (comprising fixed arrays of fluidly separated wells), which takes a significant amount of time from skilled staff. To ensure that the optimum clones are identified it is advantageous to evaluate the properties of many different clones.

Traditional mammalian cell culture techniques require performance of a series of steps (tasks), which must be carried out under aseptic conditions. The steps include: seeding transfected cells into suitable tissue culture dishes; feeding them with fresh media; expanding each clone as the cells grow and divide (for example from one well to another well with a greater available area or volume for growth); and sub-cloning to ensure that each cell line is truly monoclonal. At various stages in this process the properties of the cells are evaluated. This complete process is called the cell "lifecycle". During all steps in the process, it is critically important to maintain the unique biology of each clone and ensure that there is no accidental cross contamination of one cell line with another.

The process of generating new stable mammalian cell lines requires insertion (transfection) of DNA expressing the sequence of interest, into the DNA of an immortalised cell line. From each experiment, many thousands of potentially unique cell lines are created, each of which may have a different cell biology. Monoclonal antibody producing cells are created by the fusion of two parental cell lines, an immortal cell line and an antibody producing cell line obtained from an immunised animal. A single fusion experiment will also generate many thousands of clones, each potentially expressing an antibody with unique binding properties.

The newly generated cells may all grow at different rates, thereby increasing the complexity of the cell culture task. This is because each clone needs to be processed (e.g. fed or expanded and given more space to grow) according to its specific growth rate. It may take a number of weeks until there are sufficient cells to test, which means that all the clones must be maintained in culture until such tests are completed. It is difficult and time consuming to culture each unique cell line while still maintaining the individual properties of each, then test them to evaluate their properties. It is also the case that the process steps are unevenly distributed over time, with peak demand that exceeds the capacity to carry out the steps manually or a requirement to process cells outside normal working hours.

The difficulty of maintaining and culturing many different clones using manual methods of cell culture inevitably limits the combinations of host cell line and expression system that can be evaluated in parallel in an experiment. It is not feasible for a person to culture many hundreds or thousands of unique clones and treat each one individually. For practical reasons therefore, the size of the experiment is constrained by the numbers that can be managed, so the numbers cultured in a single experiment will generally be limited to tens or at most a few hundred. This inevitably reduces the chances of finding the optimum cell lines: either a sub-optimal cell line is selected, which can compromise the quality of experimental results obtained by using that cell line, or further experiments are performed which will take more time and effort and cause delay.

### Known cell culture systems

There is a particular requirement for the culture and maintenance of cells growing in a range of types of vessels including multiwell dishes. Use of multiwell vessels provides significantly increased capacity in support of high throughput generation and selection of stable cell lines, and other applications including provision of assay-ready plates for cell based permeability and transport studies. On the other hand, multiwell plates present challenges when performing tasks with respect to selected individual wells. This type of cell culture is time-consuming and labour-intensive when performed using conventional manual methods. Manual methods are, therefore, inherently limited in the number of cell lines that can be cultured in parallel.

Consequently, there are known cell culture systems that incorporate a degree of automation. For example, liquid handling robots have been integrated with incubators for culturing cells growing in multi-well plates. Generally this type of automation is restricted to performing repetitive process steps which treat all wells in a plate in the same way. Although it is possible for such systems to be integrated with measurement or test devices, the manufacturers do not provide the automated systems with software or control systems that make use of any measurement data. It would require a significant amount of work by the user to change or modify the software provided, so that sophisticated adjustments to cell culture conditions are made automatically in response to a measurement result, or to process individual wells in a plate selectively as dictated by the cell biology.

The applicant currently supplies products under the registered trademarks Cellmate and SelecT. Both products are examples of automated equipment for mammalian cell culture of attachment dependent cells, i.e. those that grow attached to the surface of special tissue culture vessels. Both systems are able to carry out all the tasks necessary for culture, such as seeding cells into fresh culture vessels, feeding cells, expanding cells and harvesting cells or supernatants. The systems are programmed by the manufacturer to perform the appropriate tasks automatically - and the systems are intended to carry out these tasks for hours to days without an operator present.

Although the systems are able to carry out extended sequences of tasks unattended, for several days on end, the operator must provide instructions to the system on which specific processes (e.g. feed, split, harvest) must be applied to particular cell lines. It is necessary for an operator to use their cell culture expertise both to check the cell lines, then to instruct the system on the sequence and timing, as well as the priority of all the processing steps. An operator is required to use his judgement on how to manage the robotic resources, balance the system workload, and also make sure that all cell lines are processed according to their particular cell biology. The operator's tasks of checking and instructing the machine must be repeated daily, for new cell lines with unfamiliar cell biology, or several times a week for well-characterised cell lines.

SelecT has a facility for counting cells and measuring cell viability. However only limited use is made of the data - the system adjusts the volume of media added in the next processing step, to dilute the cells to reach a pre-determined number. If an error in the cell count is detected the system stops processing. No forward planning of how cells grow is enabled on the system using cell count data.

Other types of systems for cell culture include bioreactors and fermentors for growing cells (bacteria, yeasts and mammalian) in suspension. These are frequently used at large scale (many 100s of litres) for the production of vaccines and therapeutic proteins by the pharmaceutical industry, but smaller scale systems (0.5-10 litres) are used to produce cells and proteins in research for drug discovery. Usually, these systems are designed for continuous processing and the automated steps are relatively simple. Cells are seeded into nutrient medium in the bioreactor, which may be stirred and gassed, additional nutrient media is added and waste bled off. Material (cells or supernatant) may be harvested continuously or at the end of the run, then subsequently processed for example to purify protein. If it is desired to maintain cells at a constant density then a measured volume of liquid is removed daily, and more liquid added to make up the volume.

Such bioreactors are provided with a number of monitoring systems for measurement of parameters such as: pH, CO₂, oxygen, nutrient levels, temperature and turbidity, and with means for automatically adding the required material (for pH adding acid or alkali) or other adjustments to bring the levels back into the correct range. Each fermentor vessel is effectively a single culture vessel, and generally has dedicated monitoring and adjustment equipment. A single process control system may be used to monitor and control a number of bioreactors in parallel. The limitation of this cell culture approach is that fermentors are inherently unsuited to small scale operation (of the order of tens of microlitres to tens of millilitres) and to highly parallel, complex processing tasks as is the case with many culture techniques.

There is a need for cell culture systems and techniques that are more efficient, more flexible, and less labour-intensive.

### Statement of invention

It is therefore an object of the invention to obviate or at least mitigate the above limitations and to provide a modular, automated, smart (intelligent decision making) system.

In accordance with an aspect of the invention, there is therefore provided a system for cultivating cells of a characteristic cell biology in a plurality of movable cell culture vessels, each vessel being suitable for containing cells in a culture medium, the system comprising: a liquid handling module for processing liquid material; an incubator module for maintaining the vessels in an environment suitable for cell culture; a testing module for performing measurement upon cells and/or media and generating output data; a manipulator module for conveying vessels between locations in the system; and a workflow management module for controlling the execution of processes within the system, wherein the workflow management module includes: decision making means for selectively processing cells and/or media in accordance with any of process definitions, operational rules and output data from the testing module; manipulator control means which controls the operation of the manipulator module in accordance with any of the process definitions, operational rules and decisions from the decision making means; and liquid handling control means for controlling handling operations in the liquid handling module in accordance with any of the process definitions, operational rules and decisions from the decision making means.

Both "operational rules" and "process definitions" are incorporated in the software of the workflow management module. In the following discussion, the term "process definition" applies to a description, in terms of instructions, of the actions necessary to effect a given process in the system. "Operational rules" (or "business rules") are condition-trigger rules that express the operational policy applied by the operator of the system. In other words they express, in terms of computer-interpretable rule statements, when an operation is to be triggered, what experimental priorities should govern operation and so on. Examples of such rules would govern how many cell lines are picked for further processing and what the relative priorities of different tasks were.

The system in accordance with the invention can automatically determine which process to carry out on each cell, cell line or media and when that process should be performed without needing the input of instructions from a skilled operator. The system is moreover able to determine the sequence of tasks for extended periods of time, which could be weeks, and modify the sequence, timing or nature of the task depending on measurements made on the cells (or supernatant, proteins, other cell products or components thereof) or specific cell biology together with operational or business rules. These tasks or sequences of tasks can be applied by the system selectively at the individual clone or well or culture vessel level.

Automation and the decision making facility confer a higher success rate in maintaining higher standards of production of stable cell lines, by improving the maintenance of the unique biology of cultured strains and by reducing the risk of cross-contamination. For example where there is the undesirable possibility of mixed cell populations growing in a single well (such as fibroblasts contaminating hybridomas) then the decision making and automated scheduling can ensure that unwanted cells do not overgrow the desired cells.

A further benefit of the invention is that the constraints of manual techniques, and of the limited conventional automated systems, are substantially reduced. A larger number of clones can be cultured in a single experiment, and multiple experiments can be run on one system at the same time, without needing to compromise the results of that experiment.

In protein producing implementations, the inventive system facilitates the control of processes involving many hundreds of strains of cell. Automatic determination of the sequence of tasks, such as when to harvest a particular culture and the parameters to be applied, will increase the chance of success in purifying intact protein of the right quality. The intelligent scheduling means maintains protein quality by minimising the time of exposure of the desired protein to destructive enzymes (released from lysed cells), even though the timing and sequence of tasks of protein expression is varied across many different culture vessels.

The processes for successfully identifying the conditions for protein production are comparatively less time consuming because they are more parallel than prior art production processes. Since the inventive system permits substantial reductions in the time taken to achieve production of proteins of a predetermined quantity and quality, it also removes delays in assessing whether the proteins produced are suitable for further processing.

They allow the exploration of significantly wider range of parameters and, as tasks are performed for any particular cell line, corresponding data may be stored in a database thereby providing an audit trail of the processes and parameters applied to each individual vessel or well. This audit trail function may be facilitated by the presence of bar codes on each multiwell plate in the system.

The relatively low potential chances of finding the optimum cell lines that results from the manual approach is addressed by adopting the inventive system. Smart automation of cell culture in multiwell dishes enables operators to develop cell lines more rapidly and efficiently and with a greater choice of the most suitable combination of characteristics in the selected output clones.

In a further implementation of the invention, the automated cell culture system may be used to support studies of cell differentiation. In addition it can perform all the maintenance tasks associated with provision of assay plates for a variety of cell based transport assays; these assays are an important part of the Drug Metabolism and Pharmacokinetics (DMPK) screening process. The increased transport assay capacity of the automated system, and the facility for performing assays in parallel with primary screening, allows more rapid decision making on which compounds to take forward to the next stage.

The workflow management module preferably includes scheduling means for scheduling further processes in accordance with any of the process definitions, operational rules, output data from the testing module and decisions from the decision making means.

Cell growth measurements, or measurements of desired cell biology, can conveniently be automatically scheduled throughout the lifecycle of each clone or the contents of each culture well. One benefit of scheduling is that the automation is responsive to changes in cell biology, and can schedule cell processing, such as feeding or harvest at the appropriate time determined by the cell biology.

As already mentioned, changes in cell behaviour that result from genetic modification may be unexpected, with this invention the system is able to respond appropriately to such changes without the need for operator intervention. Within a single experiment, some clones may grow faster than expected and other more slowly, and the (automated) scheduling takes account of differences across the population of cells in the experiment and modifies the future scheduled tasks appropriately.

Another benefit of scheduling, in combination with the decision making process, is the further efficient utilisation of the automation resources of the system. Two or more different experiments can be run on the same equipment without compromising either.

The workflow management module may include means for modelling cell biology.

At its simplest, modelling cell biology information ensures that cells are maintained in optimal condition, that the appropriate operations are performed on the different cells within an experiment at the correct times.

The prediction means can also be applied to scenarios where the cells, supernatant or cell products (such as protein) are ready for the operator to remove from the system for further processing. It enables staff to predict when they need to be available to interact with the system to remove samples. It ensures that other resources (such as off-line testing facilities) can be used efficiently, and that samples are in optimal condition when used.

The modelling of cell biology of a given current experiment can be compared with the results of past experiments. This provides the operator with a means to judge the progress of the present experiment, how likely it is to succeed and decide whether to continue with the experiment or discontinue.

The model can also be used to predict how cells will behave and therefore what the expected loading will be on the system at times in the future, for instance how full an incubator is.

The workflow management module advantageously includes means for examining the resulting schedule to determine system resource conflicts. The means for examining may create a requirement for labware and/or media. Alternatively or additionally, the workflow management module may be arranged to predict shortfalls in appropriate labware and/or media. The workflow management module may also be arranged to predict shortfalls in system processing capacity. Examples of this system processing capacity may be any one of: testing module measurement capacity, incubator capacity, liquid handling capacity, harvest capacity, lysis capacity, purification capacity, and centrifuge capacity.

The prediction facility is of particular importance when the shortfall in system processing capacity is a shortfall in throughput and/or space.

It is preferred that the means for examining includes means for resolving resource conflicts. The system then may include a display means, the means for resolving resource conflicts including a user interface capable of receiving operator input in order to resolve resource conflicts and representing resource conflicts on the display means.

The workflow management module may include incubator control means, which controls the operation of the incubator module in accordance with any of the process definitions, operational rules, output data from the testing module and decisions from the decision making means.

The control of liquid handling operations may include the control of the provision of liquid material delivered to or removed from a specific vessel.

The liquid handling module preferably includes a handling device for moving vessels between locations within a liquid handling area.

The liquid handling module may be capable of delivering controlled quantities of liquid material into a vessel, transferring controlled quantities of liquid material and/or removing controlled quantities of liquid material from the vessel.

In either case, the liquid handling module is preferably provided with a number of end effectors and is further arranged to pick up a selected one of the available end effectors. Preferably, the end effectors include a plurality of tip arrays, and the liquid handling device is further arranged to pick up a selected one of the available plurality of tip arrays. Alternatively or additionally, the end effectors include a piercing tool for piercing a closure.

The vessel may be provided with a lid. Consequently, the liquid handling module would be conveniently arranged to engage, remove, and/or replace the lid.

The system for cultivating cells may further comprise an input/output module for output of vessels for further use, input of new vessels containing material for processing and/or for temporary storage of clean and used vessels.

Where the system includes a plurality of cell culture vessels, each vessel being suitable for containing cells in a culture medium, and the vessels being array vessels provided with a plurality of wells, each well is preferably capable of containing a portion of liquid material in isolation from neighbouring wells. Advantageously, the system may be capable of measuring and processing cell cultures in each well of the array vessels selectively and individually, in accordance with any of the process definitions, operational rules, output data from the testing module and decisions from the decision making means.

The testing module may include an offline facility capable of receiving external data. The testing module may include an online testing apparatus for example for monitoring cell growth.

Preferably, an aseptic environment is maintained within one or more modules of the system.

### Brief Description of the Drawings

For a better understanding of the invention, reference will now be made, by way of example only, to the accompanying drawings in which:-
Figure 1 shows stages in a typical cell culture system;
Figure 2 shows a schematic diagram of a Lifecycle in the cell culture system in accordance with the invention;
Figure 3 shows a schematic flow diagram of the steps in a hybridoma process;
Figure 4 shows a schematic flow diagram of the sub-cloning stage of the hybridoma process in Figure 3;
Figure 5 illustrates the hierarchy of function, operations and sub-operations in a typical read task for execution on the inventive system;
Figure 6 shows the constituent operations and sub-operations within a plate function;
Figure 7 shows a schematic diagram of the software architecture of the workload management module;
Figure 8 shows a general process flow diagram for the workload management module in accordance with the invention;
Figure 9 shows a schematic diagram of a resource conflict handling means;
Figure 10 shows the effect of scheduling more than one read task;
Figures 11 A, 11 B 11C and 11 D illustrate the types of model used by the workflow management module;
Figures 12A and 12B illustrate the modelling of capacity over time for a number of experiments;
Figure 13 shows a plan view of a preferred arrangement of modules in a cell culture system; and
Figure 14 shows a plan view of a liquid handling module of the cell culture system in Figure 13.

### Detailed Description

### Cell Culture

Cells routinely used in drug discovery research are well-characterised. These have well-established culture requirements including controlled environment (temperature and humidity), particular nutrient media, frequency of feeding, and, in some cases, split ratio (the population of cells is divided to provide more space for growth), as well as predictable growth rates. Each laboratory will have protocols that describe the preferred methods of culture for each particular cell line.

Routine cell culture involves incubating cells at the right temperature, replenishment of the nutrient media and removal of waste material at the appropriate frequency, otherwise the cells will not thrive. It is also very important that cells are divided at the correct time. If mammalian cells have insufficient space to grow and divide, then the cell cycle is arrested and cells stop growing or may die. However, mammalian cells will not flourish if cultured at too low a density, and may require the presence of conditioned media (which has growth and other factors secreted into it from growing cells). This means that a degree of judgement, on when and how to culture any cell line, is always required from the cell culture expert, even for well-known cell lines.

Furthermore, when a cell line is genetically modified, the cell biology changes as a result of the insertion of foreign DNA, and the cells' behaviour ceases to be predictable. For example, it may be difficult to express some large membrane proteins, which therefore cause the host cells to grow much more slowly or to undergo apoptosis (programmed cell death), and such experiments can have a low success rate.

For these reasons, it is normally necessary for a skilled operator to check genetically modified cells to evaluate their progress and health - by checking their morphology for example - on a regular basis, which may be as frequently as daily. The growth rate will also determine the timing of media replenishment as well as when to divide cells. Successful generation of stable cell lines or production of protein therefore requires an advanced level of skill, experience and insight to ensure that the correct processing of the cells is carried out at the right time as the experiment unfolds. It can be appreciated that it is not easy to automate these complex tasks without incorporating the skill and knowledge of an experienced operator.

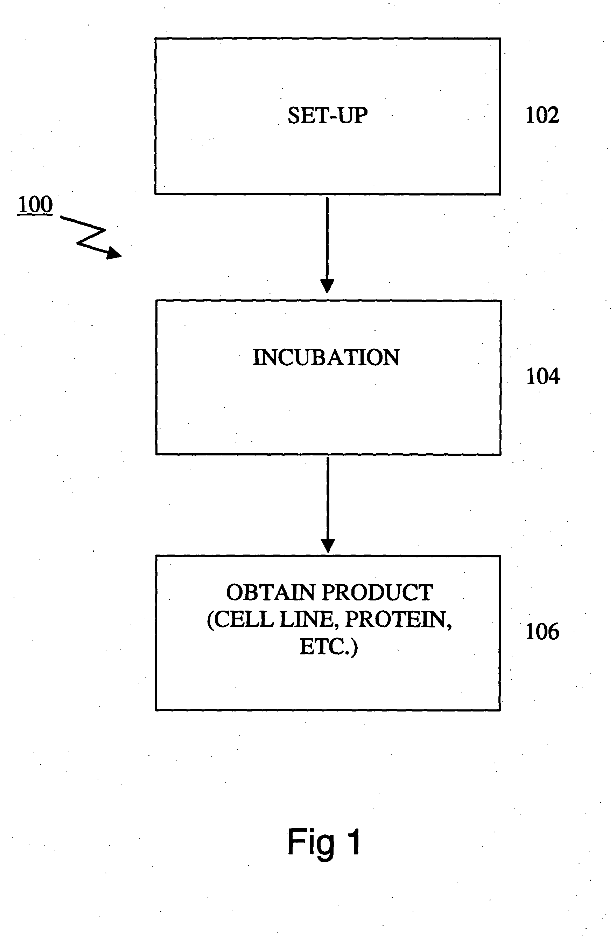

Figure 1 shows the stages fundamental to any cell culture technique 100, whether performed manually or automated. Before cell culture starts 102, the vessels must be primed with cells and a first medium.

The needs of the cultured cells are tended to in an incubation phase 104. Finally, the cultured cells are removed from incubation for storage or further processing 106 in order to obtain the desired result: either cell lines or biological factors, such as proteins.

### Incubators & Incubation

The actual cell culture takes place during the incubation phase 104. Cells are incubated in incubators that provide an environment suitable for the growth of cells (i.e. controlled temperature, CO₂ and humidity levels). Cell culture systems can be specified with one or more incubators, depending on the capacity required.

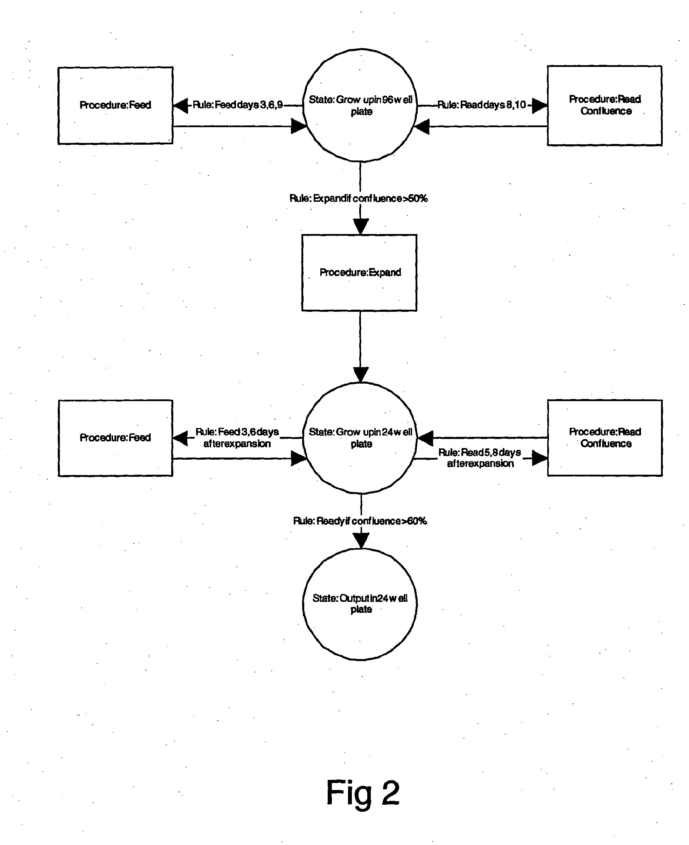

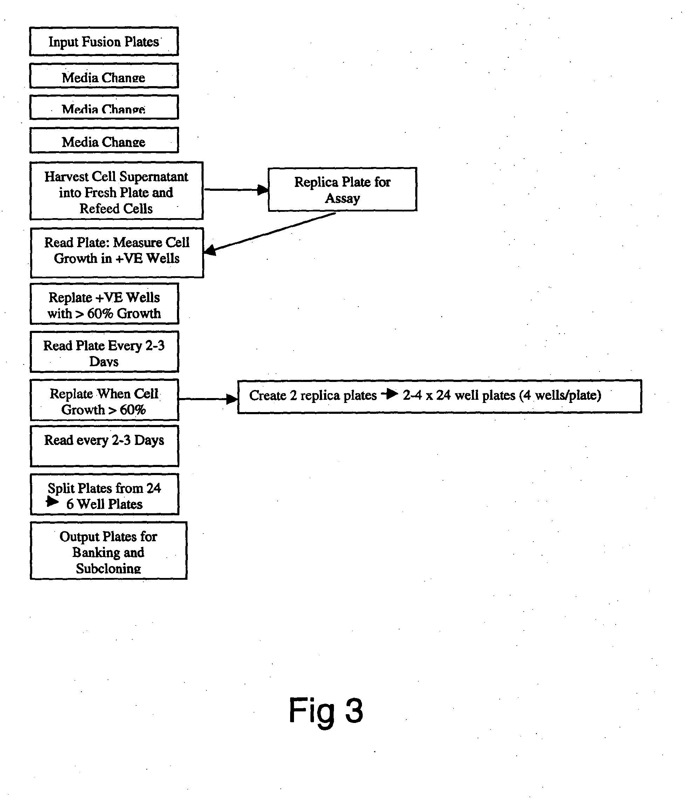

Maintaining the environment for cells as they grow requires the performance of a range of procedures. The cells need to be fed, monitored and in some cases transferred to vessels with larger volumes of media. Two representations of the sequence of steps in typical cell culture techniques are shown in Figures 2 and 3.

Adequate feeding may require full or partial replacement of the fluid media surrounding the cells.

Throughout incubation, it is desirable to monitor the state of the culture. In protein production, there is an optimum time for the introduction of the inducer: add it too early and too few cells will have matured enough to be express the desired protein ― add it too late and the cells may not express the protein. One way of determining when to dispense inducer is to measure the optical density (OD) of the cell culture. OD gives a measure of cell numbers in a vessel: the more opaque the culture, the more cells are present. By comparing this measurement with the results (i.e. protein yield) of experiments where inducer was added to cell cultures with different OD values, one can estimate whether the cell culture has matured sufficiently for the introduction of the inducer to give optimal protein production.

Another useful parameter is the "confluence" - a measure of the ratio of the surface area occupied by cells to the available surface area in cell culture medium. This is important because it measures cell crowding: if confluence is too high the potential for growth is restricted. Other parameters that may be monitored include: the numbers of colonies; cell line integrity; pH; oxygen level; and temperature.

Cell growth is generally an aerobic process so that cells also require an adequate supply of oxygen, O₂. Different cell types have different oxygen requirements: so that, for example, insect cells require comparatively less oxygen than *E. coli*.

In order to aerate the cells (to maintain cells in an appropriate form for cultivation), the cell cultures are often stirred or shaken (agitated). Different degrees of (aerating) agitation are appropriate for different cell types: insect cells require the agitation to be significantly less vigorous that *E*. *coli* cultures.

In the generation of cell lines, the cells being incubated may reach a confluence that is too high. It may be desirable to expand out the contents of each vessel into a plurality of further wells, thereby permitting the cell line to continue.

Agitation may also be necessary, before expansion, to establish the cells in homogeneous suspension (preventing the cells from settling out of solution). This may be effected through repeated sequence of aspiration and dispensing through a pipette tip.

Certain cell types are "adherent". Adherent cells cannot simply be extracted since they cling to the vessel walls. Simply agitating would destroy significant numbers of cells, so the cells must first be dissociated from the walls of the vessel and from one another. One way of dissociating the cells is to introduce an enzyme to break the bonds between cells. When the enzyme is trypsin, the dissociation process is referred to as trypsinisation.

In some protein production techniques, incubation consists of a pre-induction stage and an expression stage. Whether a pre-induction stage is necessary, and how long that stage should last, is dependent upon the cell biology. For some cell culture processes, such as the cultivation of strains of *E*. *coli*, the biology requires that the cells be grown to an appropriate maturity before they are induced to "express" a desired biological factor (protein, RNA etc.). The pre-induction stage ends when an inducer is added and expression begins.

In other cases, the cells need no pre-induction growth stage. Insect cells, for example are infected with a virus (transfection) at the onset of incubation.

Once expression is induced, the cells may have altered requirements. For example, they may require maintenance at a new temperature. In such cases, it is often desirable to acclimatise the cell cultures to the new temperature before induction.

The actual processes applied to a specific cell culture during, and after, incubation depend upon the ultimate end product. Where the end product is a monoclonal cell line, the cultured cells are themselves desired. It may, however, be necessary to ensure that the cultured cell line is monoclonal by sub-cloning from a single cell of the cell line.

On the other hand, the end product may be a protein expressed by cultured cells. Further processing actions will then be necessary to produce the desired protein. For the purposes of the following discussion, these additional processing actions include harvest, lysis and purification (HLP).

### Harvest, Lysis and Purification

HLP typically involves a sequence of centrifuging, pipetting, chemical lysis, filtration and washing actions. The cell culture is first centrifuged to bring the cells out of suspension: they form a pellet at the bottom of the centrifuge vessel. The vessel is moved to a liquid handling area, where the supernatant is aspirated (i.e. "pipetted off') leaving only the cell pellet. The walls of the cells are ruptured (lysis), preferably by dispensing a chemical lysis agent onto the pellet and agitating to ensure intermixing. The product of lysis is a suspension of cell debris and the expressed biological product. This lysis suspension is centrifuged. The cell debris settles out of the suspension leaving the biological product in suspension in the supernatant. A microwell filter plate is prepared and resin beads are introduced into each vessel in the filter plate, the beads being primed to engage the expressed biological product selectively. The vessel containing the supernatant is moved back to the liquid handling area where the supernatant is aspirated and dispensed into the microwell filter plate. The microwell plate is placed in a negative pressure assembly. A negative pressure is applied below the filter of the microwell plate to draw the supernatant through the filter (and over the beads). The biological product is bound to the resin beads. A collection plate collects the material that passed through the filter. The material caught by the filter is washed in buffer fluid. Finally, the filtered material is washed in a buffer that releases the bond between the beads and the expressed biological product (elution). The released biological product is aspirated for end processing and delivery.

### Sub-cloning

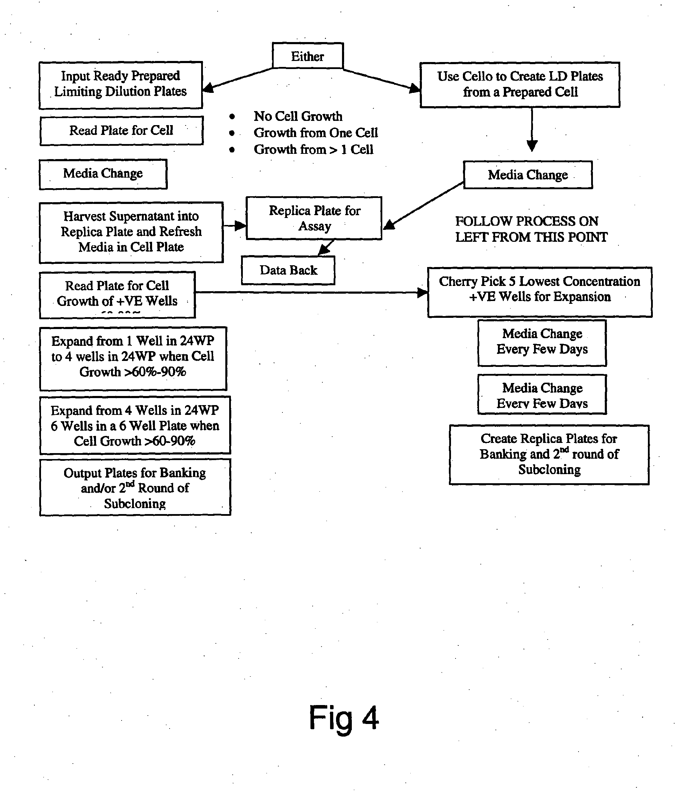

To ensure that a cell line that has been created is monoclonal, i.e. that is has been derived from one parental cell, it is necessary to perform the process of sub-cloning (see Figure 4). This process can be carried out by serially diluting cells, then seeding out the diluted solution, such that there is the equivalent of less than one cell per well. After sub-cloning, the cells are allowed to grow, and the wells must be inspected to see if there is a single, or more than one, colony of cells in each well. It is usually necessary to count or estimate how many cells are in the starting wells, so that the correct series of dilutions are performed during sub-cloning.

An alternative method of sub-cloning is to use an instrument that can automatically sort cells and deposit single cells into wells. This type of equipment is suitable for relatively large volumes of cell suspension, with volumes in the order of millilitres.

### Parallel approach

For protein production systems, it is clear that increasing the size of the experiment, that is numbers of conditions investigated in parallel, brings advantages of identifying more rapidly, and with greater certainty of success, the best set of parameters for expression of heterologous protein in bacteria, insect or mammalian cells. If more parameters and a greater range of experimental parameters are explored in a single experiment then combinations of parameters that interact in unexpected ways may also be found.

It is strategically beneficial to be able to produce specific target proteins significantly more rapidly, so that structural information on key drug targets is available when it has most impact on the discovery programme. Consequent benefits include: increased opportunities for, and efficiency of, rational drug design programmes; support for targeted compound library design and in-silico approaches; earlier access to better information to endorse decisions on target validity and "drugability"; increase in efficiency and reduction in scale of screening programmes, resulting in time and cost saving; support for programmes to co-crystallise compounds with targets related protein families, to provide information on compound specificity and early prediction of toxicity; five to ten fold improvement in time necessary to derive a protein structure; reduction from months to days in the time taken to identify an ideal protein production system, and produce high quality protein in sufficient quantity; helping to identify conditions for production and crystallisation of hitherto intractable proteins, because a larger range of multidimensional experimental space can be explored efficiently; increasing the chance of getting a drug to into clinical trials more rapidly.

In other cases, when creating stable cell lines, increasing the numbers of cell lines that are cultured in parallel in a single experiment increases the chances of finding the optimum cell line, because there is a larger population with a range of cell biologies from which to choose. A much larger population increases the chances of a finding a "rare event", that is a cell with outstanding performance. It also enables the selection of cells having a combination more than one property, such as high expressing and fast growing.

The best cell line is likely to be identified more rapidly, that is within one cell culture lifecycle and one round of experimentation, with the benefit of saving substantial amounts of time - potentially several months - and saving the resources which would be required to repeat the experiment.

Having a much larger population of clones from which to choose has many advantages. Clones with higher productivity can be selected, this gives a very significant savings during the production of therapeutic proteins both in the cost of capital equipment as well as time and materials - media etc. Furthermore, a more rapidly growing clone enables sufficient cells for a screen or assay to be produced in a much shorter time; a stable clone with the desired expression levels of the specific protein or receptor can significantly improve the results of a high throughput screening assay (the variable quality of the cell line in functional cell based screening assays is a major contributor to poor quality assay results). An antibody has properties of specificity, affinity and avidity; these are all important functional attributes contributing to its performance as a reagent in drug discovery or as a therapeutic agent. Increasing the numbers of hybridomas evaluated enables exactly the right combination of attributes to be chosen for the specific application

It will be appreciated that there is a significant challenge in managing the logistics and scheduling of processing many thousands of unique clones generated in a single experiment, in which faster and slower growing cells are at different stages. The present invention describes a system that is able to model and manage the workflow for very large numbers of clones with divergent growth while maintaining the biological diversity - which is the desired outcome of the experiment. Moreover, the present invention is able to schedule the individual tasks for a number of experiments that are progressing in parallel, each experiment consisting of hundreds or thousands of individual clones.

### Lifecycle

The procedure applied to a cell can be abstracted as a state model, referred to hereafter as a "Lifecycle". A Lifecycle specifies the complete process to be carried out on the cells in a set of input plates. A Lifecycle and the set of cells in wells it is applied to forms an Experiment.

The Lifecycle is composed of Procedures (corresponding to individual actions performed upon a cell or group of cells), States, and Rules (corresponding to triggers for Procedures and changes of State) linked together. Figure 2 shows a simple Lifecycle composed of three States shown as circles, five Procedures shown as rectangles and the Rules shown as the labelled arrows.

Procedures are the individual elements of processing done on the cells in the wells such as feeding them or cherry picking from them.

States describe the handling of cells in wells between procedures. The State specifies where the cells should be stored (for example, in an incubator at a specific temperature) and the Rules that determine the next Procedure to be applied to the cells.

Rules specify when different Procedures should be applied and to which set of items. The result of applying a Rule is a Job that consists of a Procedure and a selection of items. The internal operation of the Procedures determines the details such as the set of destination wells used for cherry picking. The operational rules are arranged to be effective when the Lifecycle is in a specific "State".

For example, consider a State called "GrowUpIn24WellPlate". The operational rules in this state might be:
- feed plate on days 3, 6, 9, 12, 15 relative to when the well entered the state
- read the plate for confluence on days 4, 6, 8, 10, 12, 14, 16
- expand any confluent wells (confluence > 60%) into 6 well plates in state "GrowUpIn6WellPlate"
- place any wells remaining after 16 days in a state "Overgrownln24WellPlate" where they are marked for disposal by the operator

Figure 3 shows a schematic flow diagram of the Procedures in a typical hybridoma process (where the end product is a monoclonal cell line). The Rules governing the triggering of the Procedures are not shown. To ensure monoclonality, at least one sub-cloning stage is required: two alternative techniques for sub-cloning are shown in Figure 4. Limiting dilution plates can either be prepared by the system from plates output in the hybridoma process (with a further check that the cell growth stems from one cell) or they can be created from a prepared cell suspension.

### Smart automation

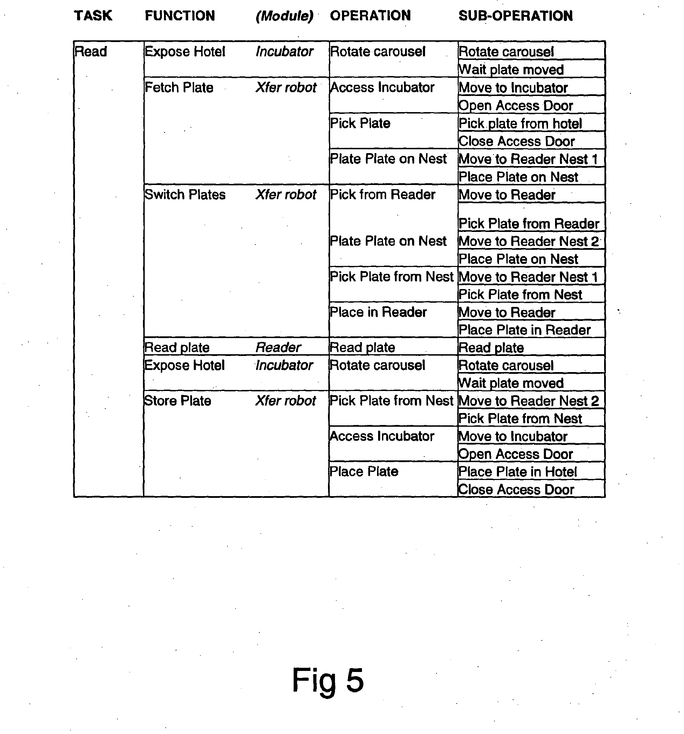

Figures 5 to 12 illustrate how the cell culture system of the invention uses hierarchical decomposition and workflow management to streamline the processing of cell Lifecycles - i.e. sequence and relative timings of processes necessary for a complete "run" through the cell cultivation stages.

In order to automate the cell culture system, a description of the actions necessary to effect each specific process must be defined in terms of executable instructions to the various components of the system. The Procedures and Rules in any Lifecycle can be viewed as a hierarchy of commands or instructions performed upon a cell. The Lifecycle for a particular cell represents the entirety of the processes carried out with respect to the cell within an Experiment in the cell culture system. The term protocol is used to describe the instruction set necessary for the cell culture system to carry out each of the tasks performed within the system, or any identifiable subset of those tasks.

A Lifecycle may comprise one or more "Stages". A Stage is a convenient level of hierarchy assigned to sets of commands to be carried out with respect to plates of a specific type. So, for example, the Procedures and Rules necessary for growing cells in 96 well plates is an example of a Stage.

Within each Stage, each Procedure that achieves a defined result, potentially using more than one of the modules in the cell culture system, is termed a "task", an example being the action of feeding cells in a plate.

It is convenient to sub-divide tasks into procedures that achieve a defined result on a specific module. The term for these procedures is "function". A function might typically define all the operations performed by a module with a vessel present at the module. An example of a function is the function that effects a change of media for a given vessel.

The process definition describes: the individual functions that the modules must perform to complete any given task; the sequence of those functions; the resources the task requires (where those resources include media, incubator locations, module processing time); how long the task takes to run; and the Rules for when other tasks of the same type can run.

Functions are hierarchically decomposed into the individual "operations" that a module has to perform to perform an individual action, often a useful action in the context of a single vessel. Examples of operations include the operations of aspirating or dispensing liquid material.

For certain purposes, it is useful to break each operation down into sub-operations. Even an operation such as aspiration relates to a number of individual sub-operations in terms of moves and actions of a robotic device. When used, sub-operations define the more detailed execution of a function.

Rules govern when or how a Procedure is to be performed. Typically, only very simple Rules apply at the level of sub-operations or operations. Rules that apply at the level of functions or tasks are often considered so fundamental to the efficient implementation of the cell culture system that they are unlikely to be changed, such as those that relate to plate geometry.

On the other hand, an experimenter might well wish to view and alter certain Rules governing the execution of the experiment. Through the script-based, hierarchical architecture, experimenters can be given access to an appropriate subset of the parameters in order that they may customise the process definition.

The combination of Procedures and fundamental Rules (which are either unalterable or have limited capacity for alteration) is generally referred to as the "process definition". Examples of such "process definitions" include a group of instructions defining when to feed cells in a particular well (e.g. every N days); a group of instructions corresponding to monitoring and performing the necessary steps that will effect the "expansion" of clones from a first generation cell culture; a group of instructions that define when samples are created for screening and how this task is to be performed; and a group of instructions that define when samples are to be banked.

The cell culture system of the present invention is delivered complete with a set of process definitions, governing the processes to be applied in any given Experiment. The process definitions are themselves assembled from empirical knowledge of the expected cell growth characteristics. Indeed, the process definitions represent a basic model of the growth of the cell types being cultivated. Process definitions take the form of scripts, a familiar concept to operators of automated devices. The scripts conform to an appropriate scripting language or protocol.

The general operational policy applied by the operator of the system is also represented as condition-triggered Rules, referred to as "business rules" or "operational rules". These Rules are however expected to be altered as required. Once set, the business rules are used in automated decision making, so that the functionality of the cell culture system can be tailored to the end user's main concerns. Broadly speaking, such Rules govern how many cell lines are picked for further processing and what the relative priorities of different tasks were.

Operational rules, too, may be embedded (i.e. provided by the manufacturer). They may also be input by the operator. As explained above, they allow the end user to express his requirements and priorities (i.e. what he wishes the system to do when, and how any process is effected). Operational rules may be provided for a range of tasks, including:
- the seeding density for newly transfected cells, and volume of cells to plate (seed) out into each well
- the type of media to use at different stages in the cell's growth
- frequency of replenishment of media, and quantity of media to use (e.g. "replace 50% of the media every 3 days")
- stage of growth governing when it is necessary to divide the cells (e.g. when a colony (group of cells) has reached a particular size; when confluence (surface area coverage by cells) has reached a particular value e.g. 70%; according to cell morphology)
- split ratio to use when dividing cells and type of cell culture vessel - surface area (e.g. 1:5 split from 96-well to 24-well culture vessel)
- duration of trypsinisation, (for example, neutralise after 5 minutes incubation)
- timing of harvest of material for testing, material to harvest (e.g. cells or supernatant), volume to harvest and output vessel type
- cell characteristics that are desirable in this experiment (e.g. protein expression level; growth rate; differentiation status - for stem cells; phenotype; and/or genotype)

Hierarchical decomposition of Procedures in combination with an easily understood process for grouping items at any level (tasks, functions, operations) means that the process definitions are very flexible. A process designer using this hierarchical decomposition has access to even the lowest levels of sub-operation and can tailor any function or task he wishes. It is noted that the expert user is able to alter process definitions (such as the liquid handling scripts), although such alterations are generally undesirable. He might do so in order to adjust the model of the cell culture intrinsic in the process definitions.

The operation of the system is arranged in such a hierarchy so that the end user need only think of the processes at the level he requires. The operator may not be interested in the particular operations performed within a particular module when he wishes to alter the timing of a "feed plate" task.

To illustrate the hierarchical decomposition used in the present invention, Figure 5 shows a typical decomposition for a read task. As may be seen, the read task is composed of functions specific to modules of a cell culture system. In the illustrated example, the read task comprises: an expose hotel function, which applies to the incubator module; a switch plate function, which applies to the manipulator robot; a switch plates function, which also applies to the manipulator robot; a read plate function, which applies to the testing module; a further expose hotel function and a store plate function which applies to the manipulator robot.

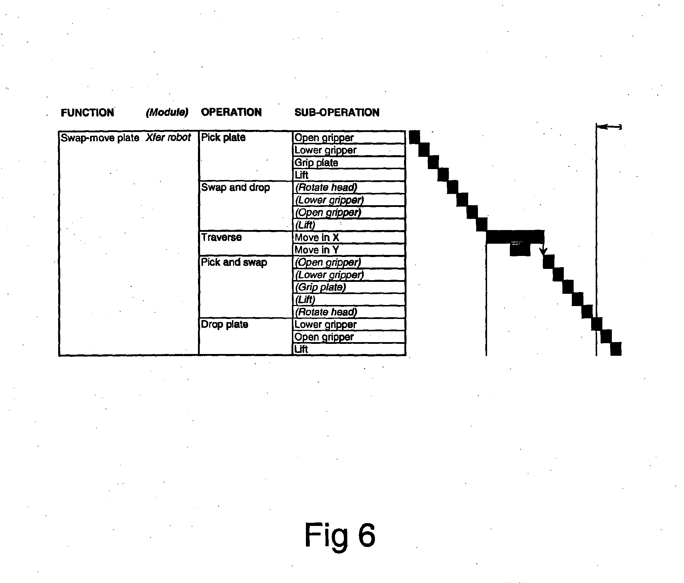

Figure 6 shows the constituent operations and sub-operations within a switch plate function. The Figure illustrates the sequencing of sub-operations and operations and the relative duration of each sub-operation.

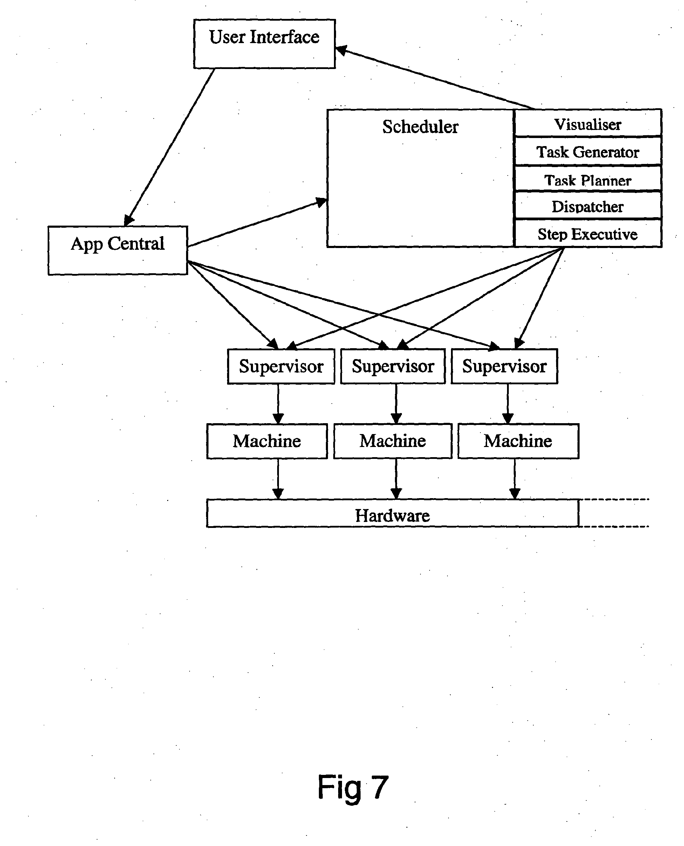

Figure 7 shows a schematic diagram of the software architecture of the workload management module. The workload management module comprises an APP central unit, a task processor, a plurality of module supervisors and a user interface.

The APP central unit controls the different modes of the management module (e.g. running, pausing, stopping etc.).

The task processor processes the process definition and operational rule scripts. The Lifecycle defined by these inputs describes what has to be done and how procedures are tied together. The feed or read actions are tasks that are made up of a number of component Procedure steps. These steps may relate to entirely different resources, thus allowing the steps to run concurrently. Once processed, these steps are passed onto the relevant module supervisor.

The task processor includes a task generator for working out from the input scripts what the system has to do and in what order. The Lifecycle provides the definitions and rules (e.g. feed every 3 days), but does not adapt to altering circumstances. The task generator outputs a sequence of tasks for execution in one or more modules, a forward planner.

The task processor illustrated performs predictive modelling, so that the task generator can look at the future and 'see' all of the tasks that will need to be completed. The task generator generates a list of Jobs, tasks in association with contents of particular vessels. To aid the task generator, the task processor also includes a task planner for mapping the tasks required against the resource available.

The illustrated task processor also includes a step executive unit and a dispatcher. As tasks are completed, the predictive model is updated with real results, in order to update the forward plan. For example, a batch of cells might be predicted to reach confluence in 10 days but might in fact only took 7 days - the forward plan would be adjusted accordingly. The dispatcher looks into what makes up each task, i.e. all the component steps required to complete it (e.g. remove lid, aspirate spent media, add fresh media etc.) and instructs the step executive unit.

A module supervisor is provided for each module in the cell culture system, i.e. for the liquid handlers, reader module, incubators etc. The module supervisor takes instruction from the task processor via the APP central unit and the step executive unit. These might be high level instructions, which it translates from the step executive unit into actions by individual modules. For example, if there is a fault, the module supervisor will inform the step executive unit that the task could not be completed due to the fault and it may receive instructions (from the step executive module) to try again.

In effect, the module supervisor ratifies an instruction before it is carried out by a particular module, e.g. it may check that the reservoirs are full before a dispense operation. Alternatively, when the step executive unit instructs the module supervisor to stop a process, the module supervisor would check to ensure the plates are lidded.

In many applications, the visualiser is optional. Where it is present, the user interface, or visualiser, allows the user to look at task generation and query whether it is appropriate. It may be used as a modelling tool, and is suitable for modelling users' processes.

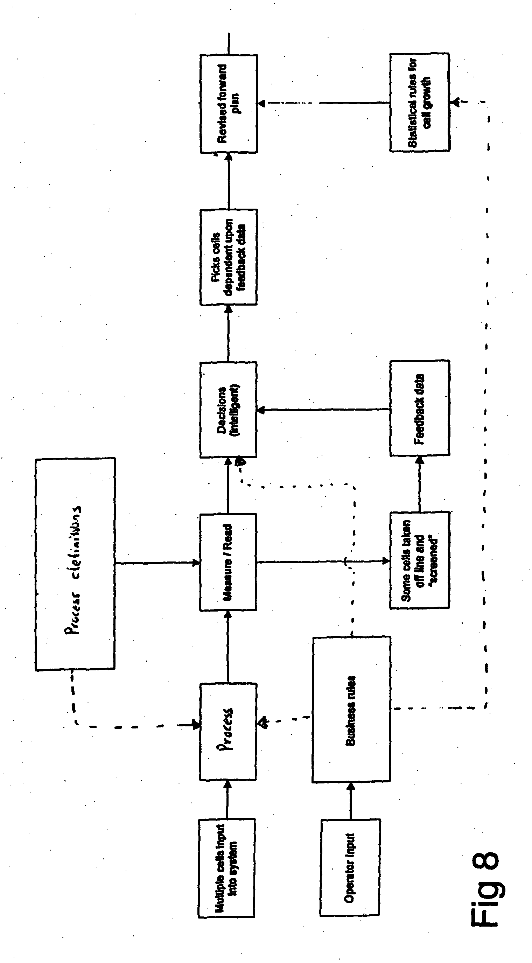

As the reader will appreciate, different experimental requirements may demand the performance of many different processes on cells in the cell culture system of the invention. Figure 8 shows a general process flow diagram for the workload management module in accordance with the invention. For any process carried out in accordance with process definitions and business rules, measurements may be taken. Using the results of these measurements (both "online" and "offline"), decisions are made as to further process steps in accordance with measured data, operational rules and process definitions.

Cells are selected for further processing in accordance with the decisions. Execution of processes within the system is controlled by: selectively processing cells and/or media in accordance with process definitions, operational rules and output data from the testing module; controlling the operation of the manipulator module in accordance with process definitions and/or decisions from the decision making means; and controlling handling operations in the liquid handling unit in accordance with process definitions and/or decisions from the decision making means.

In a preferred embodiment, the relative timings and resources for the performance of each process step are assembled in a schedule or forward plan.

The system can be provided with additional or alternative modules. The workflow management module is arranged to permit corresponding automation of the functions executed in these modules. The additional functionality can be provided by updating or extending the process definitions to define new tasks, functions, operations or even sub-operation

As in Figure 6, the individual sub-operations are generally sequenced. A sequencer may be provided to ensure that higher level Procedures (tasks and functions) follow a defined flow path and to inform the operator of requirements as and when they arise.

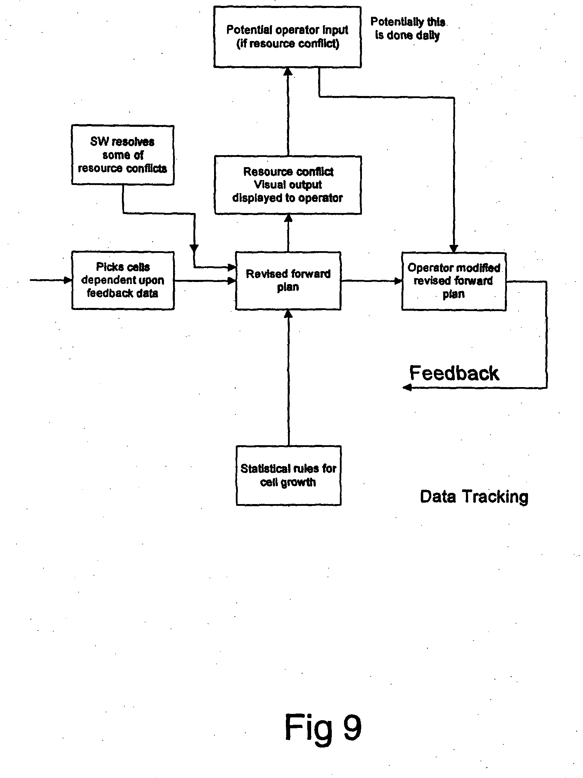

Figure 9 shows a schematic diagram of a resource conflict handling means. Resource conflict refers to situations where a requested task or group can not be carried out at the scheduled time, because of a problem with resources. Resources may be labware and/or media (there may simply not be enough of a required type of plate, or reagent). On the other hand, the resource may be less tangible, i.e. system processing capacity (the system may not have enough space or time to accommodate the planned tasks). The workflow management module may be arranged to predict shortfalls in either type of resource. The illustrated handling means includes means for resolving resource conflicts, timeslots are "shuffled" and additional consumable resources ordered in (e.g. extra 6 well plates).

To allow operator input, the illustrated resource conflict handling means includes a display means (potentially using the visualiser component and user interface of Figure 7). The user interface is capable of representing resource conflicts on the display means and receiving operator input in order to revise the forward plan, thereby manually resolving resource conflicts.

Scheduling is necessary in many cases because the available resources (modules) cannot perform all the tasks required of them simultaneously. Certain modules are inactive while other modules are busy carrying out tasks. It is possible to arrange the timing of particular tasks to take advantage of the inactive periods in each module.

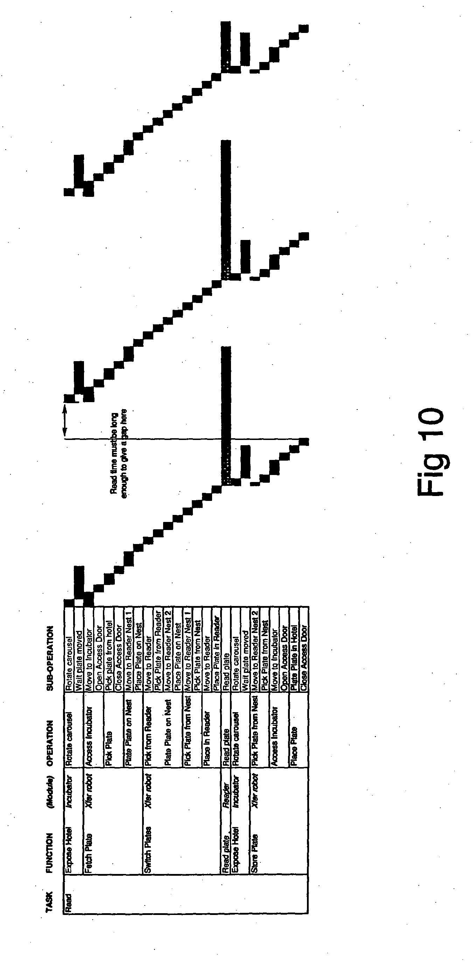

Figure 10 shows the effect of scheduling the execution of a "previous" read task 1002, a "current" read task 1004 and a "next" read task 1006. For this illustration, it will be noted that the expose hotel function applies to the incubator, while the switch plate function operates on the robot manipulator: the two functions can be (and are) performed simultaneously. Figure 10 shows the read plate function of the previous read task 1002 being scheduled to overlap with the expose hotel and fetch plate functions of the current read task 1004. The duration of the read plate function in any one read task is thus long enough to ensure that the current read task 1004 starts after the read plate function of the previous read task 1002 has completed. Scheduling ensures that functions of successive read tasks that use the same module do not overlap.

### Model

Biological processes can not be relied upon to complete in a known time. It is desirable to generate a statistical model of the biological process whereby the duration of the process can be estimated. The system conveniently incorporates such a model, thereby embodying the processes necessary to ensure that cells are maintained and kept in good condition, and that appropriate cell lines are selected. An appropriate model needs to include information that an expert operator would have, on what tasks to perform and when these need to be done. The schedule may be informed by data from a statistical model of cell growth.

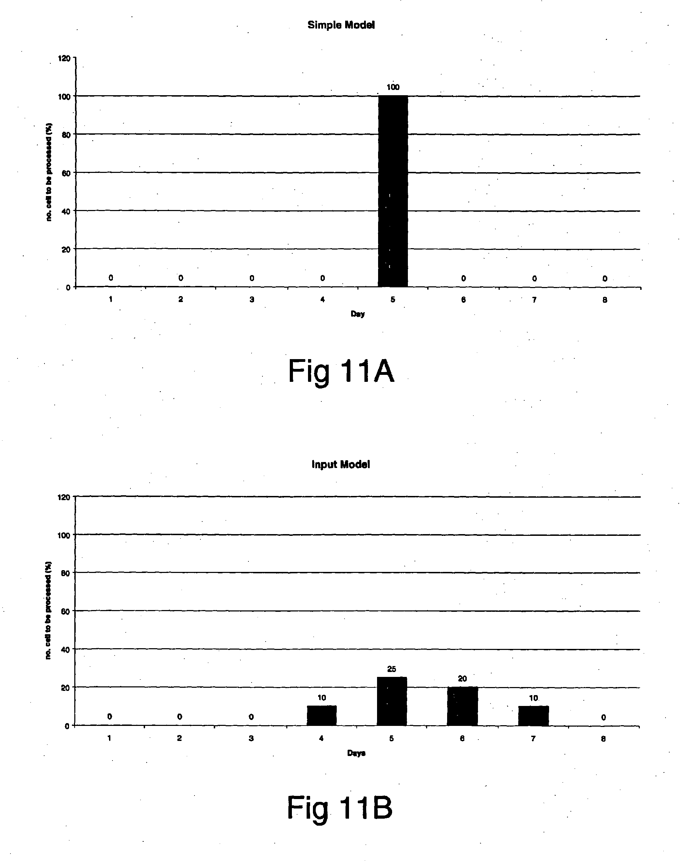

Figures 11A, 11B 11C and 11D illustrate the types of model used by the workflow management module. The "simple model" shown in Figure 11A represents the model incorporated in the process definitions. It simply assumes that all cells take exactly the same number of days to "process" (5 days) and that 100% of cells complete the process. Since the process definitions deal with all possible outcomes, this "model" is unrealistic.

An improved "input model" (as shown in Figure 11B) can be provided. The input model is a static, empirical model, which better represents what is known about the type of cell being cultured in the current Experiment. Here, the cells vary in their time to completion and a significant number fail to complete at all.

The input model need not be provided initially, provided the facility for gathering the necessary measurement details is present. With feedback from the testing module, the basic model can be updated better to reflect the modelled process.

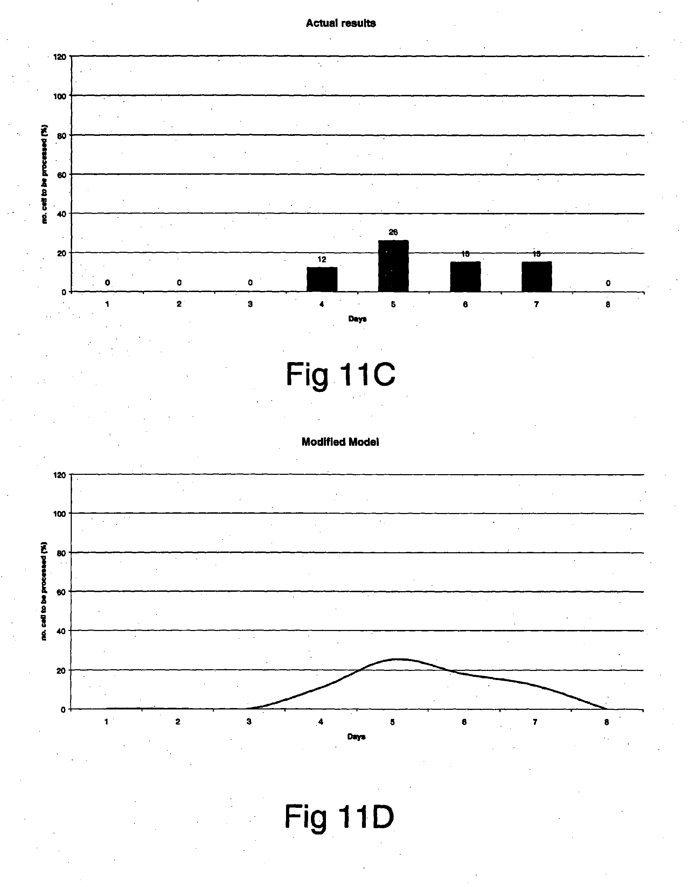

Figure 11C, representing results from an actual experiment, differs from Figure 11B. A dynamic, statistical model of the cells can be derived as a function of both the current model and the results of actual Experiments. Figure 11D represents a weighted average "modified model" derived in this way. With careful choice of functions, this model can predict the statistical spread over time of the number of colonies of cell lines at the process stage and can adapt to changes in cell characteristics.

When a (static or dynamically updated) model for how the cells will behave is incorporated in the automated system, the model can be run forwards in time to predict what the loading on the system will be at points in the future. The model allows the extrapolation from measured characteristics to the future of the current Experiment. This allows:
- the system to schedule tasks to optimise the use of the system
- the operator to make decisions about when there is capacity in the system to schedule new experiments, when to terminate experiments and when to change the number of clones taken forwards in experiments
- the operator to be informed about when samples may be ready in the future for off-line processing such as banking cell lines or off-line testing

As already explained above, cell behaviour is unpredictable. Therefore, the model is capable of being constantly changed and updated as the experiment progresses in order to predict what actions and processes are needed to maintain a healthy population of cells for any specific clone. For an experiment with hundreds or thousands of unique clones, a corresponding statistical model that is able to predict the overall behaviour of the population of clones can be used. As the experiment progresses and information about the cells is obtained by the system, then the model can be updated to incorporate the data and information derived from measurements and tests such as rate of cell growth.

Having updated the statistical model using the data measured by the system, the model can be refined to incorporate more sophisticated rules, for instance:
- to read plates to assess growth at a frequency or timing depending on how fast cells are growing
- to perform media changes depending on how fast cells are growing
- to take a sample for testing according to the number of cells in a well
- to assess the monoclonal status of a population of cells when there are sufficient numbers to count accurately, but before there are so many that the result is ambiguous
- to dilute a sample according to the number of cells in a well

Integration of the biological model with the system scheduler allows extra reading to be done when the system is lightly loaded, reducing the amount of reading that needs to be done when the system is more heavily loaded, and refining the model.

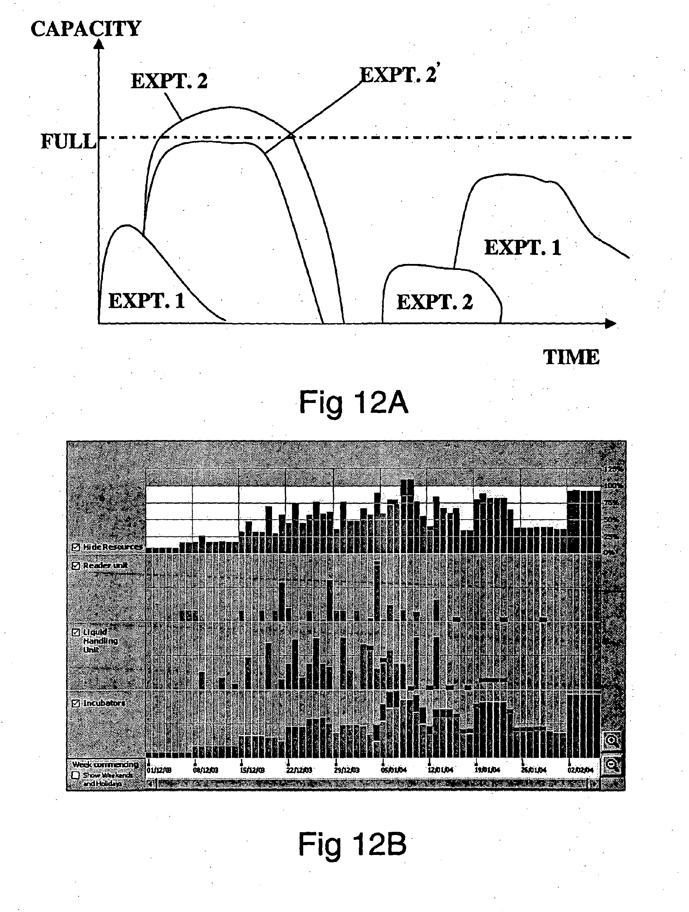

Figures 12A and 12B illustrate the modelling of capacity over time for a number of Experiments. In Figure 12A, the workload expected while both Experiment 1 and Experiment 2 are running on the system leads to a capacity shortfall (the combined requirements of the two Experiments exceed the capacity of the system). The system scheduler resolves this problem by reducing the requirements of Experiment 2 to Experiment 2'. The resulting combination of requirements can now be performed as needed.

Figure 12B illustrates the loading of different modules over time, together with a graph of the loading of the system as a whole. This Figure shows one way in which the loading data could be presented to the user on a display device.

### Operator interactions

The operator interacts with the machine on a daily, weekly and monthly basis. The daily interactions generally comprise, a short period (say 30 or 40 minutes) of interactions. However, on weekends and national holidays, the machine will not be touched by an operator. Typical interactions may include: loading new plates onto the machine; loading reagents/media onto the machine; loading wash fluids onto the machine; removing used plates for discarding or external processing; removing plates for assay; changing pipette tips; checking incubator water supply and topping up when required; loading holders with clean tips; checking the ongoing schedule for conflicts, resource needs etc. and modifying the planned work accordingly; ongoing local cleaning and maintenance of the system; transferring assay data onto the system; and, reviewing images.

The system does not expect the user to define the work to be carried out each day. The system, by means of the execution of the Lifecycle definition in the workflow management module, will already have prepared what work is to be carried out each day and, if necessary, the schedule of this work. The user can, but typically will not, override the default schedule to sort out any immediate resource conflicts, change priorities etc.

Every week the operator carries out more wide ranging maintenance and cleaning tasks. Such tasks may include: cleaning out liquid handling module reservoirs; cleaning the sealing mats on the pipette head; cleaning the tip holders; and starting new instances of Lifecycles. Typically on a six-monthly or similar basis the machine will be shutdown for maintenance.

### Embodiment

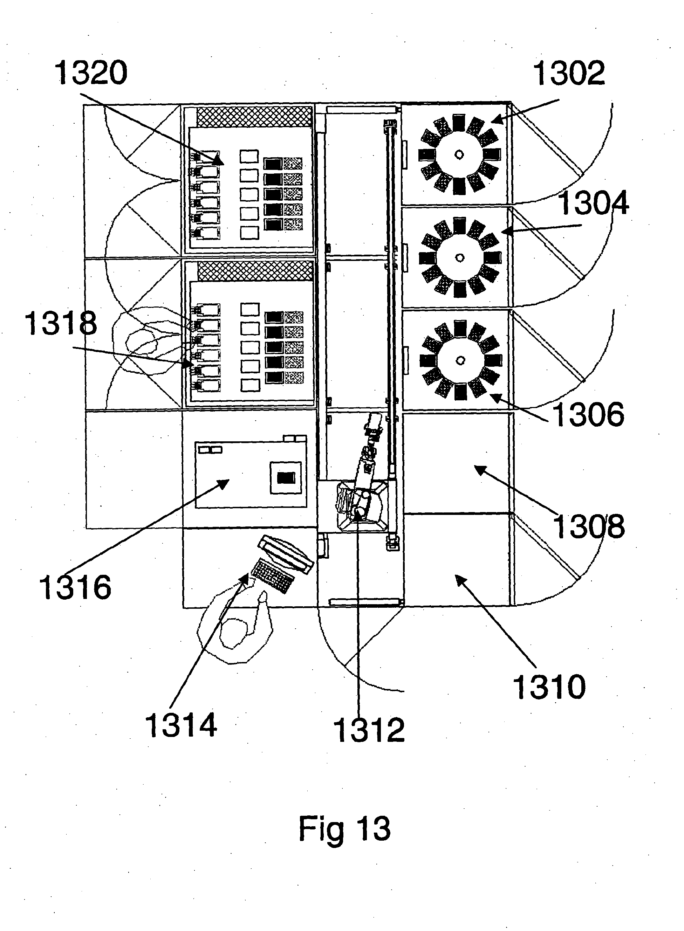

Figure 13 shows a plan view of a preferred embodiment of the invention. The system here includes two incubators 1302,1304, an input/output and storage module 1306, a testing module 1316, a manipulator module 1312,1308, two liquid handling modules 1318,1320, and a workflow management module 1310,1314.

### Incubators and I/O modules

Each of the two incubators has a capacity of a few hundred medium-depth plates (where medium depth corresponds to a depth of up to 26mm). Plates are located in batches referred to as "hotels". The hotels are, in turn, mounted in receiving positions on a rotating carousel within the incubator. All receiving positions in the incubators are equivalent and all are suitable for holding plates in a large range of standard formats. Software components of the workflow module keep sets of plates together, wherever possible, to simplify operator handling.

Each incubator is provided with an internal bar code scanner, which automatically scans the complete contents of the incubator after any operator interaction with the incubator, thereby identifying which plates have been loaded or unloaded and warning of any errors.

The incubator has an external door to allow access to the inside of the incubator for cleaning when necessary. The incubator may be provided with an inner glass door, held closed by a catch, to enable the operator to view the contents of the incubator without having to stop operation. The provision of ports allows the temperature to be checked.

In the event of an incubator failure, the operator will be able to turn the carousel by hand, and remove plates from the incubator.

In this illustrative embodiment, all operator interactions, in terms of loading and unloading plates from the system, are directed through an input/output module. The plate input/output and storage unit is used for input and output to the system as well as storage. Empty plates, (clean or used) and plates with samples (cells, protein or supernatant) for assay and plates for processing will be stored temporarily in this module. As noted earlier, the specification of the input/output and storage unit is the same as the incubators, with the same capacity and receiving positions for the same range of vessel formats.

Since plates are stored in removable hotels, the operator can load and unload plates quickly and efficiently. The workflow management module collates plate sets together to simplify loading and unloading. A paper printout may be generated to assist the user in identifying which plate hotels to remove.

The input/output and storage unit has an internal bar code reader that automatically scans all the positions in the hotels after every operator intervention, to ensure that the correct plates have been removed, and to identify where new plates have been placed.

As noted previously, the operator is responsible for loading up new plates before processing starts, and for unloading and reloading it during the day if necessary. A user interface may be provided with a consumables calculation feature to give the operator an indication of the plates (and media) needed for the next few days' processing.

For a typical industrial implementation, the loading of the incubators can reach several hundreds of plates. This loading level can be satisfied with two or more incubators. The input/output module provides additional capacity for plates that are awaiting analysis.

### Liquid handling

All liquid handling, including liquid disposal, is performed within a clean processing area of the system. In the embodiment of Figure 13, the system has two liquid handling modules, which operate in parallel. Plates spend the minimum time out of the incubator, and are returned immediately after liquid handling is completed. Only one time-critical process (e.g. trypsinisation) is performed at any one time, to ensure consistency of processing.

Two types of liquid handling may be provided: whole plate processing, where all wells in a plate are processed in parallel; and selected processing of individual wells. Whole plate processing allows efficient sampling of plates for screening or re-feeding. In a proposed embodiment, a proprietary 96-way pipetting head is used. This head automatically picks sets of tips (loaded in specially designed tip holders) from the bed of the system to perform the requested pipetting step. The tips are standard disposable pipette tips, but can ideally be washed after each use so that they can be used to process hundreds of plates before replacement.

A plurality of different tip sets can be loaded onto each liquid handling module, to accommodate different plate formats and volumes. Each tip holder will only fit into one defined location, so reducing the risk of operator error.

The preferred pipettor has fine control in x, y and z directions, which allows more complex liquid handling tasks such as moving aspirate or dispense and circular aspirate.

When re-feeding cells, aspiration and dispensing actions need to be done slowly and carefully to minimise the disturbance of cells. When changing the media, the tips follow the liquid level down as media are aspirated, both to minimise any disturbance of cells, and to reduce tip wash requirements.

The liquid handling module may include an aeration assembly for facilitating aeration functions, the aeration assembly being arranged to be moveable into the culture vessel as appropriate.

It is possible for the expert operator to optimise liquid handling parameters (by altering the appropriate process definitions), via script-based protocols. Examples of parameters that can be changed by the expert user include: aspirate and dispense speeds; aspirate and dispense height positions, and numbers of cycles to mix well contents. In this embodiment, the system is provided with software that has pre-defined volumes for each process step, but this can be changed by the normal operator when loading a new experiment.

The liquid handling system also enables the contents of a single well to be picked and transferred to a new well, so-called "cherry picking". Data from the plate testing system or from screening (together with output from the decision making means) is used by the system to select, which wells to cherry pick.

When one or more wells in a given plate need to be cherry picked, wells are processed sequentially, one at a time. Typically, each well takes a second or so to process. Once the dissociation enzyme has been added to the specific wells, the plate is placed in an incubator. After incubation, the second part of the process is also performed sequentially, aspirating the dissociated cells from each well, one after the other. The pipetting head dispenses all the picked clones simultaneously into the new plate, the plate having reagent that blocks further action of the enzyme. The overall delay between the first and last wells in a plate is in the order of a few tens of seconds. All used tips are then washed together.

Implementing a multi-channel cherry picking head in this way improves throughput both by optimising move times and reducing the numbers of wash cycles required, by washing tips in parallel.

The system typically executes a feeding task or a supernatant harvest task at a rate of about one plate a minute. Harvesting a whole plate of adherent cells with enzyme is somewhat slower. Process times depend upon plate format (6-well plates take longer to feed than 96-well plates) and numbers of clones picked per plate. Optimising overall process parameters (such as robot move speed, dispense and aspirate speeds) may also change the process times given here.

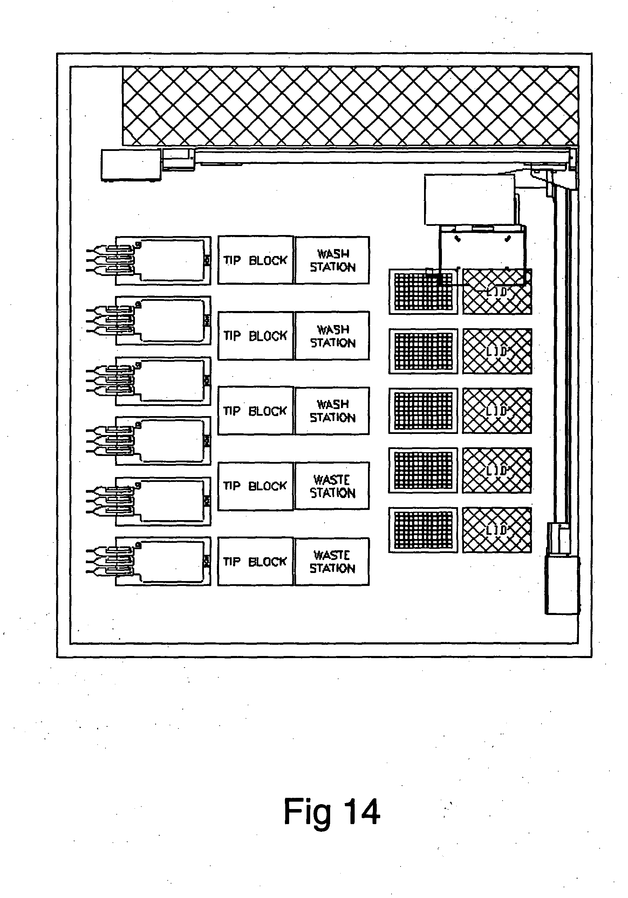

To meet the requirement for flexibility, multiple media supplies are provided. For efficient use of space, the number of media reservoirs in a liquid handling bed is limited, six reservoirs are illustrated in Figure 14. Each reservoir in the present embodiment has a volume of approximately 40ml, which is automatically topped up during processing. A level sensor feeds back the level of liquid in the reservoir to a controller, which in turn instructs the dispensing of sufficient additional liquid to raise the level. The reservoirs are removable, and can be autoclaved to sterilise them.

Certain liquid handling tasks require the handling of a larger number of different media than can be held in the available reservoirs. More than one different media can be connected to a single reservoir, where necessary. To permit the connection of many more media to the available reservoirs, each liquid handling module is preferably provided with a plurality of peristaltic pumps.

An automatic change over mechanism switches between different media supply vessels. Change-over includes automatic emptying of the reservoir and flushing through with the next reagent, to rinse away remaining material. Both the flush and refill sequences are configurable by an expert user (via alterations to the process definition). It is generally the operator's responsibility to ensure that media supplied to a single reservoir are compatible with each other. Process definitions and/or operational rules may be incorporated to ensure that this aspect is also automated.

The reservoirs are generally supplied from media containers external to the system, connected via tubing and peristaltic pumps. Bulk media are normally located at one end of the system; the tubing being routed through a cut-out in the end panel.

The system provides local storage under the bed of the liquid handling module for expensive reagents, which are used in smaller quantities, and/or for labile reagents. This minimises the dead volume in the tubing and reduces reagent wastage. The storage area can optionally be chilled to keep the reagents stored here in good condition. The numbers and volumes of containers stored here are limited by the available space.

The media reservoirs are optionally heated by circulating water, from a temperature controlled water bath, which is located under the bed of liquid handling module. The operator sets the temperature of the water bath, and manually sets which media reservoirs are warmed, the remainder are left at ambient temperature. As an alternative, a water bath or chiller unit may be supplied to cool the reservoirs. Temperature control may alternatively be automated (either computer-controlled or thermostatically controlled).

The workflow management module may be arranged to predict how much of each media is required for the planned lifecycles, and indicate when the user needs to load more consumables - plates and/or media. The prediction may also take into account the time during which the reagent is stable. During processing, the quantities of media remaining are estimated (using information supplier by the user when media is first loaded).

When the estimated volume remaining reaches a predetermined level, no further plates requiring the particular liquid are processed. An error message is generated for the user, and the system continues processing other plates that do not need that media. Similarly, if a particular plate type is unavailable, an error message is generated, and if possible the system will perform other processes that require the available plates types.

It is often acceptable to reuse the same pipette tips when processing different cell lines, provided that the reused tips are washed between each use, using 70% alcohol, to minimise the chance of cross contamination between cell lines.

Each liquid handling module is provided with associated wash stations, together with one or more waste disposal stations.

After each use the tips may be washed through a sequence of water, alcohol, water. Each tip wash station is a simple trough through which wash liquid is pumped. A level sensor controls the level of wash fluid in each wash station.

The system provides forced ventilation to ensure that the level of alcohol vapour inside the system does not build up to dangerous levels. In the event of a failure in the ventilation, power is removed from the entire system.

### Testing modules

The testing module may take a number of forms and may permit interfacing with a variety of external measurement devices. It may be advantageous to make measurements of many different aspects of the cell; such as those relating to the number and morphology of cells and colonies, cell viability, biochemistry, physiology, mRNA and DNA (both the level of and type) and specific protein production levels. Accordingly, there are many different instruments, devices and sensors that can be used to make measurements which are used by the decision making means.

Some measurements may be able to be performed non-invasively, such as cell imaging techniques, which in addition to standard microscopy may include measuring the amount and distribution of a fluorescent marker (such as green fluorescent protein) that indicates expression of a specific protein.

Other tests may require the addition of a dye or label and subsequent measurement of the labelled cell (such as Trypan blue to measure viability).

Yet other types of measurement may require taking a sample (of cells or liquids) and addition of other reagents or dyes. Both materials within the cell, and the liquid surrounding the cells may be tested in this way. The presence or level of metabolites, nutrients (including glucose, amino acids, lactate, pyruvate, nitrogen source, pH) and gases (oxygen and carbon dioxide) may be related to the health or growth of the cells or to the cells' productivity.

During the process of mammalian cell line generation, cells need to be passaged, by expansion into new wells according to how fast they have grown. A commercially available imaging system can be integrated into the system to estimate a variety of cell growth parameters.

The workflow management module uses the resulting data, the process definitions and customer-defined "business rules", to decide which process is carried out next, and its timing.

This embedded "intelligence" means that the system is able to run unattended, since the operator is not required to make decisions about the timing or processing of specific plates or wells. For example, if the degree of confluence in a well (percentage of well surface area occupied by cells) meets a threshold set by the user, then the system will automatically perform the next process (such as expansion) as defined in the cell culture protocol. The frequency at which plates are measured during the cell lifecycle and measurement type is defined as part of each specific cell life cycle.

The imaging system integrated in the Figure 3 system is preferably an automated microscope with a camera for image acquisition, together with a suite of image analysis software. Maia Scientific's (formerly known as Union Biometrica) established MIAS system is an example of an appropriate system.

The microscope may operate in bright field mode and measure plates with their lids on. A number of different types of analyses are available for a range of plate formats, they include; cell counting, confluence, colony number, colony size and "clonality" - that is whether there is a single colony or more than one colony in a well.

It is possible for the operator to view images collected by the imaging system at the same time as the system is processing cells. If necessary, the operator can review the data and/or images to confirm the results of the software analysis.

Additionally, the microscope hardware may be upgraded to enable fluorescent measurements to be made on plates. The microscope is preferably able to swap between different measurement modes automatically.

In this embodiment, the system can work with a range of morphologies, for example: epithelial, fibroblast and suspension cell morphologies.

The measurement times for testing procedures are typically of the order of a few minutes. These times are somewhat dependent upon the number of wells in each plate and, in confluence testing procedures, upon whether the full well or only a quadrant is tested. Clearly, cell counting and clonality testing take longer the more wells there are.

It should also be noted however that measurement times are very dependent on plate quality - plates with flat wells improve the speed of the autofocus operation. Poorer quality plates can take up to twice as long to image as high quality plates, and 6-well plates can be problematic.

The estimated reading times mean that it is often important to schedule when plate reading occurs during each lifecycle, to ensure that reading time does not become rate limiting on overall system throughput.

In addition to making measurements of all wells in a plate, the system is able to take measurements from only selected wells in a plate, where appropriate, which will decrease plate read time. The workflow management module directs the imaging system as to which measurement type is used and the specific wells to measure.

Depending on the user's preference, and at any stage in the cell "lifecycle", the measurement can be made at different levels of accuracy. A representative portion of the well can be imaged (quadrant measurement), which is quicker but potentially of lower accuracy. Alternatively, the total well area can be measured for greater accuracy. Partial images can be measured acentrically, that is a portion of the well periphery is included; in principle this should be more representative of the well overall than an image taken only from the centre of the well.

### Further modules

Depending upon the particular implementation of the cell culture system, further modules may be incorporated in the system. There may, for example, be a requirement for a centrifuge module (as would be the case in the HLP stage of protein production).

The system illustrated in Figure 13 also includes a manipulator module (also referred to as a transfer robot). The transfer robot (1312) moves plates between plate incubators, storage carousel, plate testing system and the clean processing area with its associated processing bed, according to the protocol selected for those cells at that stage in their lifecycle. In the event of an electrical power failure, or emergency-stop (E-stop) or compressed air failure the robot gripper will maintain its current position so that the plate is not dropped.

The modules of the illustrated system interconnect to form a system enclosure. The system enclosure or housing has a number of functions: it acts as a physical safety guard protecting staff from the materials being processed and from the operation of the robot and other process stations, it also provides controlled air quality to the plate processing area and rest of the enclosure. Each liquid handling module will have its own air handling system, with fans and high efficiency particulate air (HEPA) filters to give clean, laminar airflow where plate processing occurs. The system will have air flow indicators, and if the flow falls below the set level, then an alarm will go off, but the system will continue processing. Pre-filters may be provided before the HEPA filters but are not considered necessary.

The enclosure will have glazed panels to allow personnel to view the system in operation. Access to the enclosure for set-up, cleaning, operation and maintenance is via a number of doors. The doors are interlocked to ensure operator safety.

The system is preferably arranged to be fumigated using a process based on hydrogen peroxide. Either the whole system or individual modules can be fumigated. As noted earlier, blanking plates may be provided to cover the module openings during fumigation and these blanking plates would have ports for connection to the fumigation equipment. Some openings may require taping to achieve an effective seal during fumigation.

The system is controlled through an operator interface using a monitor (VDU), keyboard and mouse. This is built into the system and positioned so that an operator working at the keyboard can view the plate processing area. The system has a worktop area of sufficient size to accommodate the display, keyboard and mouse.

The operator will use the VDU to check progress, to view cell images from the microscope, to view the contents of the input/output carousel and incubators. A printer is provided to create print outs to guide the operator on the location of plates to remove for assay.

As noted earlier, an important part of the user interface is the forward view of system utilisation. This is displayed module by module, and show how busy the different modules are predicted to be, giving a forward view of several weeks. From this, the user is able to identify any potential resource conflicts - e.g. too much work in the time available on that module - and is able to reschedule processing to minimise the impact of this.

The system further includes a means of exchanging data, for example importing information about new plates of cells and exporting data on samples for screening. The system may conveniently use XML format files for data exchange. As an alternative the operator is able to input data via the user interface.

Where XML format files are used, XML schema may be defined for registration and import of new plates of cells, export of samples for screening, import of screening results etc. At the start of an experiment, when plates with cells are registered into the system, data on their contents is provided in XML files. These files contain for example: plate bar code; ID of the cells in each well; and other information about the project, job etc.

When the system exports samples in plates for screening, for example for clone checking or for assay, it will also export the corresponding plate-map data in an XML file. This file may be loaded into a database or other tool as required. This file will contain for example: plate bar code; ID of the cells from which each sample is derived; and date and time of export.

Results of screening experiments may likewise be returned in an XML file. This file will contain for example: plate bar code; and pass/fail for each well

The system is provided with a flexible reporting tool, which allows the user to extract ad-hoc data from its internal database. Extracted data may be cut and pasted into Microsoft Excel [RTM] for further processing or exported, as an XML file, for loading into another system.

Selected image data, relating to plates output from the system, can be placed into a local or remote data storage, for later off-line analysis or archiving. An XML file linking the data to the corresponding plate/well may also be written. The system will preferably store images locally for approximately one week, before deleting them - oldest first.

If multiwell plates are created in an appropriate manner, the associated data may be imported directly into the system. This means that the operator only needs to load the hotels of plates, the data includes information on which plates contain which cells, and therefore the system automatically processes each plate using the correct lifecycle.

To achieve this, the system for creating plates and the cell culture system are ideally linked to the same computer network. The link is ideally a local connection. Archiving image data and providing a back up of the database is enabled by connection to a suitable file server. In a preferred implementation, the system is supplied with a RAID card.

Typical processing rates for different processing tasks are in the order of tens of plates processed in an hour. The most efficient way to use the system is to have an even spread of workload. This is generally achieved by loading multiple, smaller batches onto the system through the week, rather than loading large numbers of plates infrequently - which can lead to subsequent bottlenecks in cherry picking or plate imaging.

### Error handling

To maintain a high level of reliability, the system is designed wherever possible to retry operations to avoid stopping the system as a result of transient errors. All retries are logged, so that any underlying problem can be addressed. Moving plates is the most critical function within the system. The system is therefore arrayed to tolerate the failure of the sensors on the robot gripper moving the plates.

In preferred implementations, the software also has built-in diagnosis, error recovery and fault handling routines. Error and warning messages are logged and displayed at the user interface. A fault indicator light and audible alarm may be provided for alerting the operator that the system needs attention. Examples of warning messages requiring attention would include: when new labware supplies are low; when liquid supplies are low; or when plates are waiting to be removed.

Each module of the system operates independently and a fault with one module will only directly affect processing using that module. When the system runs out of a consumable, e.g. media, the system will return the plates affected to the incubators and continue to perform other processes. Where plates have been partially processed by the system, the fact is flagged for the operator.

It is possible to connect to the user interface via modem to determine what actions need to be taken and when these actions are required. The user interface need not be a local connection. Notifications may be sent by electronic messaging (e.g. email, text message, media message). The operator can thus receive these electronic communications wherever he has access to a communications network (either wired or wireless). It is preferred that the electronic communication may be sent to a remote PC, a personal digital assistant (PDA) or a suitable mobile handset, for example a Blackberry device.

## Claims

1. A system for cultivating cells of a characteristic cell biology in a plurality of movable cell culture vessels, each vessel being suitable for containing cells in a culture medium, the system comprising:
a liquid handling module (1320,1318) for processing liquid material;
an incubator module (1302,1304) for maintaining the vessels in an environment suitable for cell culture;
a testing module (1316) for performing measurement upon cells and/or media and generating output data;
a manipulator module (1312) for conveying vessels between locations in the system; and
a workflow management module (1310,1314) for controlling the execution of processes within the system, wherein the workflow management module includes:
decision making means for selectively processing cells and/or media in accordance with any of process definitions, operational rules and output data from the testing module;
manipulator control means which controls the operation of the manipulator module in accordance with any of the process definitions, operational rules and decisions from the decision making means; and
liquid handling control means for controlling handling operations in the liquid handling module in accordance with any of the process definitions, operational rules and decisions from the decision making means.

2. A system for cultivating cells as claimed in claim 1, wherein the workflow management module includes scheduling means for scheduling further processes in accordance with any of the process definitions, operational rules, output data from the testing module and decisions from the decision making means.

3. A system as claimed either of claim 1 or claim 2, wherein the workflow management module includes means for modelling cell biology.

4. A system as claimed in claim 2, wherein the workflow management module includes: means for examining the resulting schedule to determine system resource conflicts.

5. A system as claimed in claim 4, wherein the means for examining creates a requirement for labware and/or media.

6. A system as claimed in either of claim 4 or claim 5, wherein the workflow management module is arranged to predict shortfalls in appropriate labware and/or media.

7. A system as claimed in either of claim 4 or claim 5, wherein the workflow management module is arranged to predict shortfalls in system processing capacity.

8. A system as claimed in claim 7, wherein the system processing capacity is any of: testing module measurement capacity, incubator capacity, liquid handling capacity, harvest capacity, lysis capacity, purification capacity, and centrifuge capacity

9. A system as claimed in either of claim 7 or claim 8, wherein the shortfall in system processing capacity is a shortfall in throughput and/or space.

10. A system as claimed in any one of claims 4 to 9, wherein the means for examining includes means for resolving resource conflicts.

11. A system as claimed in claim 10, wherein the system further comprises a display means and wherein the means for resolving resource conflicts includes a user interface capable of receiving operator input in order to resolve resource conflicts and representing resource conflicts on the display means.

12. A system as claimed in any one of the preceding claims, wherein the workflow management module includes incubator control means which controls the operation of the incubator module in accordance with any of the process definitions, operational rules, output data from the testing module and decisions from the decision making means.

13. A system as claimed in any one of the preceding claims, wherein the control of liquid handling operations includes the control of the provision of liquid material delivered to or removed from a specific vessel.

14. A system as claimed in any one of the preceding claims, wherein the liquid handling module includes a handling device for moving vessels between locations within a liquid handling area.

15. A system as claimed in claim 13, wherein the liquid handling module is capable of delivering controlled quantities of liquid material into a vessel, transferring controlled quantities of liquid material and/or removing controlled quantities of liquid material from the vessel.

16. A system as claimed in either of claim 14 or claim 15, wherein the liquid handling module is provided with a number of end effectors and is further arranged to pick up a selected one of the available end effectors.

17. A system as claimed in claim 16, wherein the liquid handling module is provided with a plurality of tip arrays, and wherein the liquid handling device is further arranged to pick up a selected one of the available plurality of tip arrays.

18. A system as claimed in any of claims 14 or 17, wherein the liquid handling module is provided with a means for piercing a closure.

19. A system as claimed in any one of claims 13 to 16, wherein the vessel is provided with a lid and the liquid handling module is further arranged to engage, remove, and/or replace the lid.

20. A system for cultivating cells as claimed in any one of the preceding claims, further comprising an input/output module for output of vessels for further use, input of new vessels containing material for processing and/or for temporary storage of clean and used vessels.

21. A system as claimed in any one of the preceding claims, wherein the system includes a plurality of cell culture vessels, each vessel being suitable for containing cells in a culture medium, the vessels being array vessels provided with a plurality of wells, each well being capable of containing a portion of liquid material in isolation from neighbouring wells.

22. A system as claimed in claim 21, wherein the system is capable of measuring and processing cell cultures in each well of the array vessels selectively and individually, in accordance with any of the process definitions, operational rules, output data from the testing module and decisions from the decision making means.

23. A system as claimed in any of the preceding claims, wherein the testing module includes an offline facility capable of receiving external data.

24. A system as claimed in any of the preceding claims, wherein the testing module includes an online testing apparatus for monitoring cell growth.

25. A system as claimed in any of the preceding claims, wherein an aseptic environment is maintained within one or more modules of the system.
